# EUROPEAN PATENT SPECIFICATION

(11) **EP 1 485 031 B1**
(45) Date of publication and mention of the grant of the patent: **13.05.2009**
(21) Application number: 03727618.5
(22) Date of filing: 17.03.2003
(51) Int. Cl.: A61B 17/17

(54) **A SURGICAL NAVIGATION TOOL**
CHIRURGISCHES NAVIGATIONSWERKZEUG
UN INSTRUMENT DE NAVIGATION CHIRURGICAL

(30) Priority: 15.03.2002 GB 0206131
(43) Date of publication of application: 15.12.2004
(73) Proprietor: Depuy International Limited, Beeston, Leeds LS11 8DT (GB)
(72) Inventor: ASHBY, Alan, York YO30 6LN (GB); REVIE, Ian, Boroughbridge Y051 9AX (GB)
(74) Representative: Belcher, Simon James
(86) International application number: PCT/GB2003/001157
(87) International publication number: WO 2003/077775

(56) References cited:
- DE-U- 29 703 947
- US-A- 4 891 516
- US-A- 5 569 247

## Description

It can be necessary in surgical procedures to drill a hole in a bone. The drilled hole might be used to fasten an implanted component or an instrument to the bone. It can be important that the location and orientation of the drilled hole is accurately controlled, for example to avoid damage to surrounding tissue. It can also be important to ensure proper positioning (for example location and alignment) of the component or instrument that is to be attached to the bone.

It is known for example to attach implanted components to a patient's spinal column using screws which are inserted into holes drilled in the pedicle. Such components can be used for example to restrict or to prevent relative movement between vertebrae. The use of screws has advantages over other fixation techniques, for example using hooks, because of the greater control that is available over the location of the components, both during and immediately after the surgical procedure, and also in the longer term. However, accurate location of the hole in the pedicle is fundamentally important in order to avoid damage to CNS tissue.

It is common to plan a surgical procedure using images of the patient's tissue, for example a CT image. This can be used to identify the site for fixation of an implant or an instrument, including for example the location and orientation of holes that have to be drilled in a patient's bone. It is necessary then to move from the CT image data to locate the fixation site relative to the bone itself. In the case of fixation to a patient's spinal column, it is an established technique to test the location of a fixation site by forming a pilot hole in the pedicle (for example using a drill bit or a pedicle probe). The hole can then be probed to ensure that the pedicle wall has not been penetrated. However, accurate location of the hole relative to the patient's bone can require generation of image data, for example using X-ray or fluoroscopy techniques.

In one aspect, the present invention provides an instrument for locating an axis on or in relation to which an orthopaedic surgery procedure is to be performed, which comprises:
a. a column member comprising upper and lower parts of which the lower part can be fastened to a bone, the upper part having a mating surface at its lower end which defines an upper part mating plane and the lower part having a mating surface at its upper end which defines a lower part mating plane, the upper and lower parts being connected to one another with the mating surfaces in contact with one another in such a way that the upper and lower parts can be rotated relative to one another about an axis which is perpendicular to the mating planes, the axis being non-parallel to the axes of the upper and lower parts of the column member,
b. a guide having an opening extending through it, which is fastened to the upper part of the column member so that it expends transversely relative to the upper part of the column member and can be moved relative, to the upper part of the column member so as to change the distance between the opening and the point at which the guide is fastened to the upper part of the column member.

The tool of the present invention has the advantage that it allows an axis to be located accurately relative to a patient's bone accurately and simply. The two parts of the column member provide a simple mechanism for determining the orientation of the axis, and the movement of the guide relative to the upper part of the column member allows selection of the distance of the axis from the column member.

Preferably, the guide comprises a plate which has at least first and second opening extending through it, the distance from the point at which the plate is fastened to the upper part of the column member to the first opening being greater than the distance from the point at which the plate is fastened to the upper part of the column member to the second opening. For example, the plate can have a plurality of openings extending through it, arranged on a helical line around the point at which the plate is fastened to the upper part of the column member. The distance from the said fastening point to the closest opening is preferably not more than about 15 mm, more preferably not more than about 10 mm.

Preferably, the distance from the said fastening point to the opening which is furthest from the fastening point is at least about 20 mm, more preferably at least about 25 mm, especially at least about 30 mm.

The plate can have the configuration such as would result from it being formed from a continuous sheet of material. However, the plate can have other configurations, for example comprising a plurality of spokes in which the openings are provided. The spokes can be formed from a continuous sheet of material. However, the plate can be formed from more than one piece of material: for example, when the plate comprises a plurality of spokes, the spokes can be provided by different pieces of material which are joined together to form the plate.

The guide component of the instrument can be an arm which has an opening in it. Preferably, the length of the arm can be varied, for example by means of two telescoping parts. The length of the arm can be controlled by means of a driver which moves the parts relative to one another, for example by means of a stepping motor.

Preferably, the guide can be rotated relative to the upper part of the column member, about the point at which the guide is fastened to the upper part of the column member. It is particularly preferred that the tool includes a motor for rotating the guide relative to the upper part of the column member.

Preferably, each of the mating surfaces of the upper and lower parts of the column member is planar. However, the surfaces might be non-planar, for example to make the engagement of the upper and lower parts of the column member more secure. For example, one of the mating surfaces might have a recess formed in it, and the other of the mating surfaces might have a protrusion which can fit into the recess. The mating planes that are defined by the surfaces will generally be defined by the edges of the surfaces, and will be perpendicular to the axis about which the parts rotate relative to one another.

Preferably, the angle between the axis of relative rotation and the axis of the upper part of the column member is the same as the angle between that rotation axis and the axis of the lower part of the column member. Preferably, the angle between the axis of relative rotation and the axis of the lower part of the column member is at least about 5°, more preferably at least about 10°, for example about 15°.

Preferably, the height of the column member is at least about 25 mm, more preferably at least about 40 mni, especially at least about 60 mm.

Preferably, the instrument includes a motor for rotating upper part of the column member relative to the lower part of the column member.

Preferably, the instrument includes a connector by which the lower part of the column member can be fastened to a bone. For example, the connector can comprise clamp parts which are configured to fit on to bone structures. Alternatively, the connector can be fitted to wires which are fastened to bone structures, for example by insertion into the spinal process in the case of a spinal application. Suitable clamp parts are known, for fitting to a patient's spinal column or to another bone structure. The lower part of the column member can be attached to the connector, preferably so that rotation of the lower part of the column member relative to the bone, around the axis of the lower part of the column member, is permitted. Preferably, the instrument includes a motor for rotating the lower part of the column member relative to the bone.

Preferably, the instrument of the invention includes at least one marker by which the tool can be located relative to the patient's bone. Generally, the instrument will include a plurality of markers, for example two, three or four markers. These can be used to locate the instrument relative to anatomical landmarks on the patent's bone, or relative to markers which have been implanted in the patient's bone. The nature of the markers will depend on the nature of the monitoring system which is being used to locate the instrument. For example, the markers can be selected to be visible using fluoroscopic techniques, or the instrument can have a shadow which is uniquely identifiable in the fluoroscopic image.

Preferably, the guide component of the instrument is made from a radio-translucent material so that it does not prevent the creation of images of the patient's bone. Other components of the instrument can also be made from radio-translucent material. However, it can be preferred for other components to be made from metals in some cases. Examples of suitable metals might include certain stainless steels and titanium-based alloys.

Preferably, one or more motors which are incorporated in the instrument are controlled using appropriate computer apparatus. For example, the motors might be stepping motors, controlled by appropriate pulse drive signals which are generated by a computer.

In another aspect the invention provides a navigation system for locating an axis on or m relation to which an orthopaedic surgery procedure is to be performed, which comprises a navigation tool as discussed above. The system can include other components. For example, it can include a cutting tool which can be used to cut the bone along an axis that is determined by the navigation tool. The cutting tool can be a drill. The cutting tool can fit through the opening in the guide when appropriately aligned relative to a patient's bone, to drill a hole in the bone. Alternatively, a separate guide can be positioned using the navigation tool of the invention, which is then used to align a.drill or other cutting tool.

The navigation system of the invention can include a computer which is used to control the navigation tool, for example to provide drive signals to motors by which components of the navigation tool are moved relative to one another. The navigation system can include sensors for determining the location of the navigation tool.

Embodiments of the present invention will now be described by way of example with reference to the accompanying drawings, in which:
Figure 1 is a side view of the navigation tool of the present invention.
Figure 2 is a plan view of the tool shown in Figure 1.
Figure 3 is an isometric view of a clamp which can be used to fasten the navigation tool to a patient's spinal column.
Figure 4 is a view of the navigation tool of the present invention positioned in relation to a patient's spinal column so as to enable the axis for a drilling step into the pedicle to be defined:

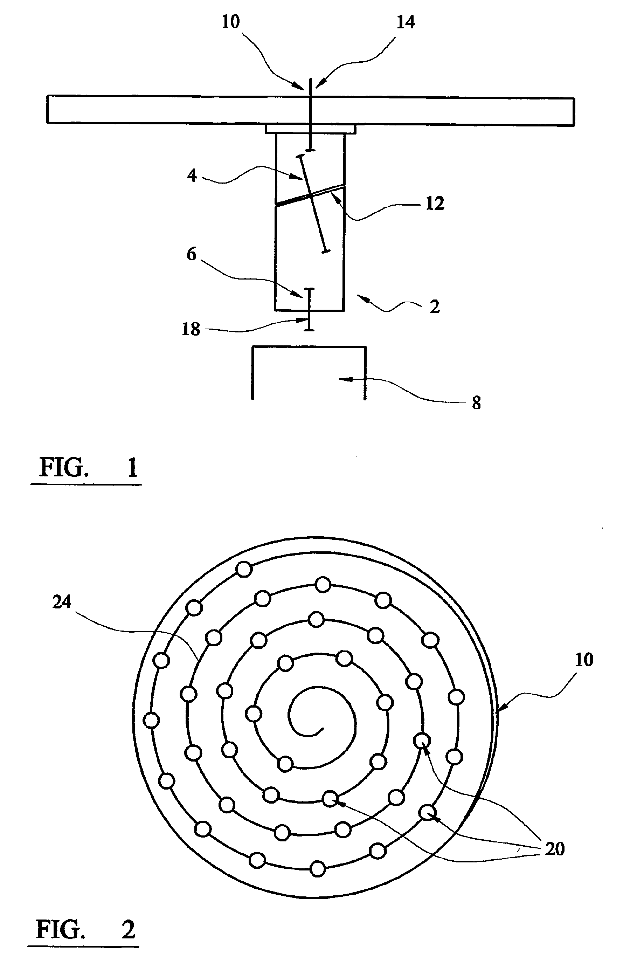

Referring to the drawings, Figure 1 shows a navigation tool 2 which comprises a column member having an upper part 4 and a lower part 6. The lower parts 6 can be fastened to a clamp 8 which can be fitted to a patient's bone.

The tool 2 includes a guide plate 10.

The upper part 4 of the column member has a lower face which defines a mating plane 12, which is inclined to the axis 14 of the upper part at an angle of 15°. Similarly, the lower part 6 of the column member has an upper face which is inclined to the axis 18 of the lower part an angle of 15°. The lower face of the upper part and the upper face of the lower part are in face-to-face contact with one another on the mating plane.

The tool includes a guide plate 10 which is fastened to the upper part 4 of the column member at (or towards) its upper end. As shown in Figure 2, the guide plate has a series of openings 20 extending through it; arranged on the surface of the guide plate on a line 24 which follows a helical path around the centre of the plate.

The lower part 6 of the column member is able to rotate relative to the clamp 8. The guide plate 10 is able to rotate relative to the upper part 4 of the column member. The upper part 4 of the column member is able to rotate on the mating plane 12 relative to the lower part 6. Relative rotation between the upper and lower parts of the column member causes the angle between the axes 14, 18 to change, between 0° as shown in Figure 1 and 30° when the upper part has been rotated relative to the lower part through 180°. Motors are provided in the column member by which the relative rotation of adjacent parts of the navigation tool can be controlled. Signals for driving the motors in the column member can be generated using a computer having appropriate software. For example, when the motors are steeping motors, the control system can generate pulse signals to drive the motors.

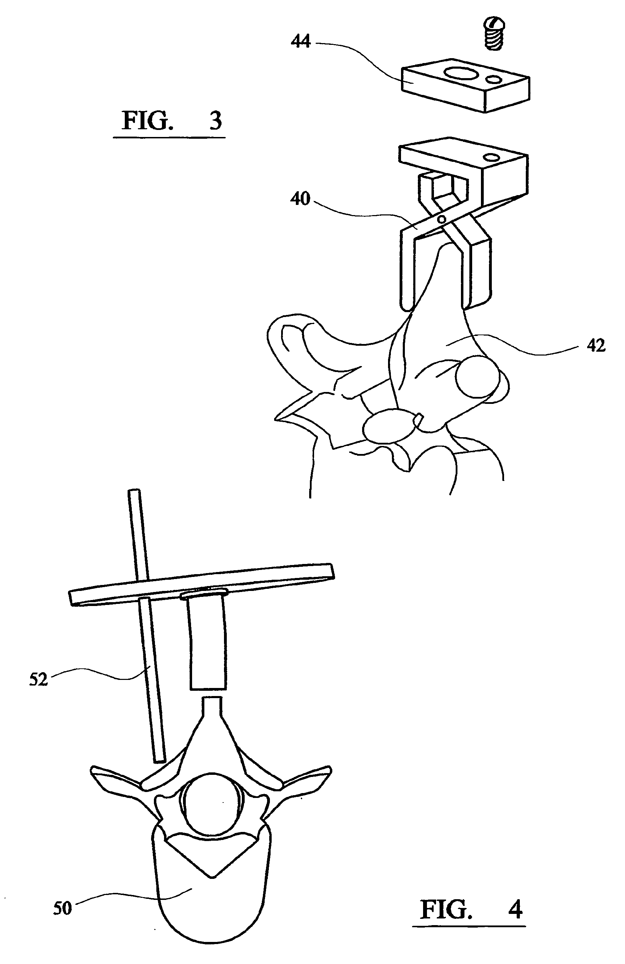

Figure 3 shows a clamp 40 which is fastened to a patient's vertebra 42, and which provides a platform 44 on which the tool of the invention can be mounted. The precise form of the clamp is not important to the function of the tool. The clamp should provide a secure mounting platform which is located stably relative to the bone. Such clamps are known.

Figure 4 shows a vertebra 50 viewed along the spinal axis which has a navigation tool according to the present invention held in relation thereto in order to allow a drilling axis 52 to be identified so that a hole can be drilled into the patient's pedicle.

Use of the system of the present invention to define the axis for drilling into a patient's bone (for example the spinal pedicle) involves the following sequence of steps:
1. The location of a drill axis is determined in pre-operative planning using appropriate images of the patient's bone, so that the location of the axis is known relative to reference points on the patient's bone (which can be provided by implanted markers or by anatomical landmarks).
2. A small incision is made to allow access to the patient's bone.
3. The clamp for the navigation tool is fastened to the bone, for example by directly engaging the bone tissues, or by means of previously fixed wires.
4. The tool is positioned in a reference configuration.
5. The location and orientation of the tool is determined relative to implanted markers or anatomical landmarks on the patient's bone using appropriate inspection or scanning instrumentation. Eor example, a fluoroscopic imaging system could be used to generate two calibrated, stereoscopic, synchronized images to identify the location of the drill guide and the bone to which it is attached in 3 dimensional space. The trajectory of the drill can then be planned and the resultant axis and direction of the guide programmed from this plan. The orientation of the guide could then be positioned as described below.
6. Drive signals are supplied to the navigation tool of the invention, involving relative movement between (a) the upper and lower parts of the column member, (b) the lower part of the column member and the clamp, and (c) the upper part of the column member and the guide plate, so that one of the openings in the guide plate is positioned and oriented appropriately to define a drill axis extending into the patient's bone, consistent with that defined in the pre-operitive planning.
7. A drill is used extending along the axis defined by the opening in the guide member to form a bore in the bone.

## Claims

1. A navigation tool (2) for locating an axis on or in relation to which an orthopaedic surgery procedure is to be performed, which comprises:
a. a column member comprising upper and lower parts of which the lower part (4) can be fastened to a bone, the upper part (6) having a mating surface at its lower end which defines an upper part mating plane (12) and the lower part having a mating surface at its upper end which defines a lower part mating plane, the upper and lower parts being connected to one another with the mating surfaces in contact with one another in such a way that the upper and lower parts can be rotated relative to one another about an axis which is perpendicular to the mating planes, the axis being non-parallel to the axes of the upper and lower parts of the column member,
b. a guide (10) having an opening (20) extending through it, which is fastened to the upper part of the column member so that it extends transversely relative to the upper part of the column member and can be moved relative to the upper part of the column member so as to change the distance between the opening and the point at which the guide is fastened to the upper part of the column member.

2. A tool as claimed in claim 1, in which the guide comprises a plate which has at least first and second openings extending through it, the distance from the point at which the plate is fastened to the upper part of the column member to the first opening being greater than the distance from the point at which the plate is fastened to the upper part of the column member to the second opening.

3. A tool as claimed in claim 2, in which the plate has a plurality of openings extending through it, arranged on a helical line around the point at which the plate is fastened to the upper part of the column member.

4. A tool as claimed in claim 1, in which the guide can be rotated relative to the upper part of the column member, about the point at which the guide is fastened to the upper part of the column member.

5. A tool as claimed in claim 4, which includes a motor for rotating the guide relative to the upper part of the column member.

6. A tool as claimed in claim 1, which includes a motor for rotating upper part of the column member relative to the lower part of the column member.

7. A tool as claimed in claim 1, which includes a connector by which the lower part of the column member can be fastened to a bone.

8. A tool as claimed in claim 7, in which the connector permits rotation of the lower part of the column member relative to the bone, around the axis of the lower part of the column member.

9. A tool as claimed in claim 8, which includes a motor for rotating the lower part of the column member relative to the bone.

10. A tool as claimed in claim 1, which includes at least one marker by which the tool can be located relative to the patient's bone.

11. A navigation system for locating an axis on or in relation to which an orthopaedic surgery procedure is to be performed, which comprises a navigation tool as claimed in claim 1, and a cutting tool which can be used to cut the bone along an axis that is determined by the navigation tool.

## Patentansprüche

1. Navigationswerkzeug (2) zum Auffinden einer Achse, auf der oder in Bezug zu der eine orthopädische Operationsprozedur ausgeführt werden soll, welches aufweist:
a. ein Säulenelement, das einen oberen und einen unteren Teil aufweist, wobei der untere Teil (4) an einem Knochen festgelegt werden kann, der obere Teil (6) eine Passfläche an seinem unteren Ende hat, die eine Passebene (12) des oberen Teiles definiert, und der untere Teil eine Passfläche an seinem oberen Ende hat, die eine Passebene des unteren Teils definiert, wobei der obere und der untere Teil miteinander mit den Passflächen in Kontakt zueinander in einer solchen Weise verbunden sind, dass der obere und der untere Teil relativ zueinander um eine Achse gedreht werden können, die senkrecht zu den Passebenen ist, wobei die Achse nicht parallel zu den Achsen des oberen und des unteren Teiles des Säulenelements ist,
b. eine Führung (10) mit einer Öffnung (20), die sich durch sie erstreckt, die an dem oberen Teil des Säulenelementes so befestigt ist, dass sie sich quer in Bezug auf den oberen Teil des Säulenelementes erstreckt und relativ zu dem oberen Teil des Säulenelementes bewegt werden kann, um so die Entfernung zwischen der Öffnung und dem Punkt, an dem die Führung an dem oberen Teil des Säulenelementes festgelegt ist, zu ändern.

2. Werkzeug nach Anspruch 1, bei dem die Führung eine Platte aufweist, die wenigstens eine erste und eine zweite durchgehende Öffnung hat, wobei die Entfernung von dem Punkt, an dem die Platte an dem oberen Teil des Säulenelementes festgelegt ist, zu der ersten Öffnung größer ist als die Entfernung von dem Punkt, an dem die Platte an dem oberen Teil des Säulenelementes festgelegt ist, zu der zweiten Öffnung.

3. Werkzeug nach Anspruch 2, bei dem die Platte eine Vielzahl von durchgehenden Öffnungen hat, die auf einer Spirallinie um den Punkt angeordnet sind, an dem die Platte an dem oberen Teil des Säulenelementes befestigt ist.

4. Werkzeug nach Anspruch 1, bei dem die Führung in Bezug auf den oberen Teil des Säulenelementes, um den Punkt, an dem die Führung an dem oberen Teil des Säulenelementes befestigt ist, gedreht werden kann.

5. Werkzeug nach Anspruch 4, das einen Motor zum Drehen der Führung in Bezug auf den oberen Teil des Säulenelementes umfasst.

6. Werkzeug nach Anspruch 1, das einen Motor zum Drehen des oberen Teiles des Säulenelementes in Bezug auf den unteren Teil des Säulenelementes umfasst.

7. Werkzeug nach Anspruch 1, das einen Verbinder umfasst, mit dem der untere Teil des Säulenelementes an einem Knochen festgelegt werden kann.

8. Werkzeug nach Anspruch 7, bei dem der Verbinder die Drehung des unteren Teiles des Säulenelementes in Bezug auf den Knochen um die Achse des unteren Teiles des Säulenelementes erlaubt.

9. Werkzeug nach Anspruch 8, das einen Motor zum Drehen des unteren Teiles des Säulenelementes in Bezug auf den Knochen umfasst.

10. Werkzeug nach Anspruch 1, das wenigstens einen Markierer aufweist, mit dem das Werkzeug in Bezug auf den Knochen des Patienten geortet werden kann.

11. Navigationssystem zum Auffinden einer Achse, auf der oder in Bezug auf die eine orthopädische Operationsprozedur ausgeführt werden soll, das ein Navigationswerkzeug nach Anspruch 1 und ein Schneidwerkzeug, das verwendet werden kann, um den Knochen entlang einer Achse zu schneiden, die durch das Navigationswerkzeug festgelegt ist, aufweist.

## Revendications

1. Instrument de navigation (2) pour situer un axe sur lequel ou en relation avec lequel une intervention chirurgicale orthopédique doit être réalisée, comprenant :
a. un élément en colonne comprenant des parties supérieure et inférieure dont la partie inférieure (4) peut être fixée à un os, la partie supérieure (6) ayant une surface d'appariement au niveau de son extrémité inférieure qui définit un plan d'appariement de la partie supérieure (12) et la partie inférieure ayant une surface d'appariement au niveau de son extrémité supérieure qui définit un plan d'appariement de la partie inférieure, les parties supérieure et inférieure étant reliées l'une à l'autre avec les surfaces d'appariement en contact l'une avec l'autre de telle sorte que les parties supérieure et inférieure puissent tourner l'une par rapport à l'autre autour d'un axe qui est perpendiculaire aux plans d'appariement, l'axe étant non parallèle aux axes des parties supérieure et inférieure de l'élément en colonne,
b. un guide (10) ayant une ouverture (20) s'étendant dans celui-ci, qui est fixé à la partie supérieure de l'élément en colonne de sorte qu'il s'étende transversalement par rapport à la partie supérieure de l'élément en colonne et puisse être déplacé par rapport à la partie supérieure de l'élément en colonne de façon à modifier la distance entre l'ouverture et le point au niveau duquel le guide est fixé à la partie supérieure de l'élément en colonne.

2. Instrument selon la revendication 1, dans lequel le guide comprend une plaque qui a au moins des première et deuxième ouvertures s'étendant dans celle-ci, la distance du point auquel la plaque est fixée à la partie supérieure de l'élément de colonne à la première ouverture étant supérieure à la distance du point auquel la plaque est fixée à la partie supérieure de l'élément en colonne à la deuxième ouverture.

3. Instrument selon la revendication 2, dans lequel la plaque a une pluralité d'ouvertures s'étendant dans celle-ci, disposées sur une ligne hélicoïdale autour du point au niveau duquel la plaque est fixée à la partie supérieure de l'élément en colonne.

4. Instrument selon la revendication 1, dans lequel le guide peut tourner par rapport à la partie supérieure de l'élément en colonne, autour du point auquel le guide est fixé à la partie supérieure de l'élément en colonne.

5. Instrument selon la revendication 4, qui comprend un moteur pour faire tourner le guide par rapport à la partie supérieure de l'élément en colonne.

6. Instrument selon la revendication 1, qui comprend un moteur pour faire tourner la partie supérieure de l'élément en colonne par rapport à la partie inférieure de l'élément en colonne.

7. Instrument selon la revendication 1, qui comprend un connecteur par lequel la partie inférieure de l'élément en colonne peut être fixée à un os.

8. Instrument selon la revendication 7, dans lequel le connecteur permet la rotation de la partie inférieure de l'élément en colonne par rapport à l'os, autour de l'axe de la partie inférieure de l'élément en colonne.

9. Instrument selon la revendication 8, qui comprend un moteur pour faire tourner la partie inférieure de l'élément en colonne par rapport à l'os.

10. Instrument selon la revendication 1, qui comprend au moins un marqueur par le biais duquel l'instrument peut être situé par rapport à l'os du patient.

11. Système de navigation pour situer un axe sur lequel ou en relation avec lequel une intervention chirurgicale orthopédique doit être réalisée, qui comprend un instrument de navigation selon la revendication 1 et un instrument tranchant qui peut être utilisé pour couper l'os le long d'un axe qui est déterminé par l'instrument de navigation.
